# EUROPÄISCHE PATENTANMELDUNG

(11) **EP 2 474 238 A1**
(43) Veröffentlichungstag der Anmeldung: **11.07.2012**
(21) Anmeldenummer: 11150454.4
(22) Anmeldetag: 10.01.2011
(51) Int. Cl.: A23L 1/221, A23L 1/224, A23L 1/225, A23L 1/30, A61K 36/82, A61K 36/67, A61K 36/48

(54) **Nahrungsergänzungsmittelpräparat mit einer Wirksubstanzmischung**

(71) Anmelder: PM-International AG, 1618 Luxembourg (LU)
(72) Erfinder: Schmitt, Gerhard, Dr., 64625 Benzheim (DE); Fritzmeier, Franz, Dr., 91710 Gunzenhausen (DE)
(74) Vertreter: Zech, Stefan Markus

(57) **Zusammenfassung**

Nahrungsergänzungsmittelpräparat mit einer Wirksubstanzmischung, wobei die Wirksubstanzmischung Trigonella foenum-graecum L. (Bockshornkleesamen), Camelia sinsensis mit einem Anteil von Polyphenolen und Coffein, ayurvedisches Gewürzextrakt, insbesondere Pfefferextrakt sowie ein stimmungsaufhellend wirkendes, Scharfstoffe enthaltendes Agens mit Bestandteilen aus Zwiebel und Senf enthält.

## Beschreibung

Die Erfindung betrifft ein Nahrungsergänzungsmittelpräparat mit einer Wirksubstanzmischung, wobei die Wirksubstanzmischung enthält: Trigonella foenum-graecum L. (Bockshornkleesamen), camelia sinsensis mit einem Anteil von Polyphenolen und Coffein, ayurvedisches Gewürzextrakt, insbesondere Pfefferextrakt sowie ein stimmungsaufhellend wirkendes, Scharfstoffe enthaltendes Agens mit Bestandteilen aus Zwiebel und Senf nach den Merkmalen von Patentanspruch 1.

Diätische Lebensmittel bzw. diätische Nahrungsergänzungsmittel, um Übergewicht entgegenzuwirken bzw. durch Einnahme über einen bestimmten Zeitraum hinweg Übergewicht zu reduzieren, sind in unterschiedlichen Ausgestaltungen bekannt. Der Abbau von Übergewicht bzw. die Reduzierung von Übergewicht ist jedoch meist ein langwieriger Prozess, der auch mit Genussverzicht und Rückschlägen verbunden sein kann. Teilweise wiederum führt Niedergeschlagenheit zum so genannten Frustessen, so dass etwaige bereits erreichte Diäterfolge auch schnell wieder zunichte gemacht werden können.

Ausgehend von dieser Ausgangssituation stellt sich die vorliegende Erfindung die Aufgabe, ein Nahrungsergänzungsmittelpräparat zu schaffen, das einen nachhaltigeren und zuverlässigeren Diäterfolg ermöglicht.

Diese Aufgabe wird gelöst mit einem Nahrungsergänzungsmittelpräparat nach den Merkmalen des Anspruches 1. Vorteilhafte Weiterbildungen sind in den Unteransprüchen angegeben.

Ein Kerngedanke der vorliegenden Erfindung besteht dabei darin, ein Nahrungsergänzungsmittelpräparat zu schaffen, bei dem mehrere Wirkmechanismen ineinander greifen und das eine Wirksubstanzmischung mit folgenden Bestandteilen umfasst:
- Trigonella foenum-graecum L. (Bockshornkleesamen),
- Camelia sinsensis mit einem Anteil von Polyphenolen und Coffein,
- Ayurvedisches Gewürzextrakt, insbesondere Pfefferextrakt sowie
- ein stimmungsaufhellend wirkendes, Scharfstoffe enthaltendes Agens mit Bestandteilen aus Zwiebel und Senf.

Trigonella foenum-graecum L. (Bockshornkleesamen) kann für sich allein verschiedene Wirkmechanismen hervorrufen, nämlich zunächst im Magen aufquellen und dadurch allein schon ein Gefühl der Sättigung hervorrufen. Der Wirkmechanismus zwischen Magen und Gehirn beruht dabei auf entsprechenden nervalen Verbindungen und erfolgt über das vegetative Nervensystem. Zusätzlich kann Trigonella foenum-graecum L. (Bockshornkleesamen) eine humorale Einwirkung auf das Hungerempfinden, insbesondere eine Dämpfung des Hungerempfindens auslösen. Schließlich kann Trigonella foenum-graecum L. (Bockshornkleesamen) Zucker im Magen und Darm binden und so dessen Resorption verzögern, so dass der Zuckerspiegel nicht steil ansteigt und insofern weniger Insulin ausgeschüttet wird. Die Reduktion des Insulingehaltes im Blut bewirkt wiederum eine bessere Fettverbrennung. Bekanntermaßen initiiert die Lipase die Fettverbrennung, wobei Insulin die Lipase bzw. Blutlipase blockiert.

Camelia Sinsensis ist beigefügt vorrangig wegen des hohen Anteils an Polyphenolen sowie an Coffein. Die Polyphenole unterstützen die Thermogenese sowie die Fettverbrennung. Das Coffein kann als Initiator zur Fettverbrennung angesehen werden. Es unterstützt die Energiebereitstellung und damit ebenfalls den Fettabbau.

Die Beimengung des ayurvedischen Gewürzextraktes, insbesondere Pfefferextrakt, stärkt die Resorption der Wirksubstanzmischung generell und unterstützt ebenfalls den Fettabbau.

Erfindungsgemäß ist schließlich ein stimmungsaufhellendes, Scharfstoffe enthaltendes Agens mit Bestandteilen aus Zwiebel und Senf beigemischt.

Mit der so geschaffenen Wirksubstanzmischung wird ein Diäterfolg in wesentlich nachhaltigerer und zuverlässigerer Weise sichergestellt. Einerseits werden verschiedene physiologische Prozesse angestoßen bzw. unterhalten, um eine Gewichtsreduzierung zu erreichen, andererseits wird dabei gleichzeitig sichergestellt, dass eine positive Grundstimmung aufrechterhalten bleibt, so dass die Diät nicht als Einschränkung der Lebensfreude empfunden wird. Weiterhin wird das Risiko von Diätdurchbrechungen erheblich reduziert, da die mit dem Nahrungsergänzungsmittelpräparat nach der vorliegenden Erfindung unterstützte Diät nicht bzw. weniger stark als Einschränkung bzw. Entbehrung verstanden wird.

In einer bevorzugten Ausgestaltung ist das stimmungsaufhellend wirkende, Scharfstoffe enthaltende Agens in einem Anteil von 2 Gew.% bis 20 Gew.%, vorzugsweise in einem Anteil von 4 Gew.% bis 15 Gew.% in der Wirksubstanzmischung enthalten.

Das stimmungsaufhellend wirkende, Scharfstoffe enthaltende Agens mit Bestandteilen aus Zwiebel und Senf kann ein oder mehrere Bestandteile der folgenden Stoffe oder Pflanzen bzw. Stoffe oder Extrakte aus diesen Pflanzen aufweisen: Zwiebel (Allium cepa) und/oder Senf (Sinapsi albis). Dadurch wird wie nachfolgend dargelegt, eine stimmungsaufhellende Wirkung durch die Beeinflussung körpereigener hormoneller Regelkreise erreicht.

Es ist bekannt, dass Zwiebel deutliche Mengen an Serotonin und Katecholamin enthält [Kimura M: Fluorescence histochemical study on serotonin and catecholamine in some plants: The Jap J Pharmacol 18(2) 162-168 (1968)]. Dies kann ihre bekannte Wirkung als Antidepressivum erklären [Sakakibara H, Yoshino S, Kawai Y, Terao J: Antidepressant-like effect of onion (Allium cepa I.) powder in rat behavioural model of depression; Biosc Biotechnical Biochem 72(1) 94-100 (2008]. Die Wirksubstanzen der Zwiebel, die Scharfstoffe Sinigrin und Sinalbin, die auch in Senf vorkommen, erhöhen aber auch den Serotoninspiegel [Sakakibara H, Yoshino S, Kawai Y, Terao J: Antidepressant-like effect of onion (Allium cepa I.) powder in rat behavioural model of depression; Biosc Biotechnol Biochem 72(1) 94-100 (2008)]. Der Effekt beruht offenbar auf einer Wiederaufnahmehemmung des Serotonins. Serotonin hemmt auch direkt die NMDA-Aktivität [Zong P, Yuen EY, Yan Z: Modulation of neuronal excitability by serotonin-NMDA interaction in prefrontal cortex; Mol Cell Neurosci 38 (2) 290-299 (2008)].

Zusätzlich kann die Wirksubstanzmischung auch noch Ingwer enthalten. Ingwer enthält ebenfalls Scharfstoffe, die ähnlich wie Bestandteile von Zwiebel und Senf eine stimmungsaufhellende Wirkung herbeiführen. In Ingwer ist 1,4 Cineol für den stimmungsaufhellenden Effekt verantwortlich: ERV, de Sousa DP, de Vasconcelos SMM, de Franca-Fonteles MM, de Sousa FCF: Anxiolytic-like effect of monoterpene 1,4-cineol in mice; Pharm Biochem Behav 96 (3) 287-293 (2010).

In einer möglichen Ausgestaltung weist das Nahrungsergänzungsmittelpräparat Trigonella foenum-graecum L. in einem Anteil von 15 Gew. % bis 90 Gew. %, vorzugsweise in einem Anteil von etwa 65 Gew. % bis 80 Gew. % in der Wirksubstanzmischung auf.

In einer ebenfalls konkreten, bevorzugten Ausgestaltung ist in der Wirksubstanzmischung Camelia sinsensis mit einem Anteil von 5 Gew. % bis 50 Gew. %, vorzugsweise mit einem Anteil von 20 Gew. % bis 30 Gew. % enthalten.

In einer weiteren möglichen Ausgestaltung ist in der Wirksubstanzmischung das ayurvedische Gewürzextrakt, insbesondere der Pfefferextrakt, mit einem Anteil von 0,05 Gew. % bis 8 Gew. %, vorzugsweise mit einem Anteil von 0,15 Gew. % bis 0,4 Gew. % enthalten.

Die Wirksubstanzmischung kann in einer bevorzugten Ausgestaltung weiterhin Curcumaextrakt und/oder Bitterorangenextrakt enthalten. Die Beimischung von Curcumaextrakt kann eine Appetitzügelung bewirken. Bitterorangenextrakt kann ebenfalls zur Gewichts- bzw. Körperfettreduktion beitragen, da es positiv auf die Thermogenese einwirkt.

Sofern Curcumaextrakt enthalten ist, erscheint ein Anteil von 2 Gew. % bis 2,5 Gew. %, vorzugsweise ein Anteil 0,7 Gew. % bis 1,3 Gew. % innerhalb der Wirksubstanzmischung als vorteilhaft.

Sofern Bitterorangenextrakt enthalten ist, erscheint ein Anteil von 0,2 Gew. % bis 4,0 Gew. %, vorzugsweise ein Anteil von 0,6 Gew. % bis 2,0 Gew. % in der Wirksubstanzmischung als vorteilhaft.

Das Nahrungsergänzungsmittelpräparat kann insbesondere für eine Bereitstellung in Kapseln so konfektioniert sein, dass die Tagesdosis für Trigonella foenum-graecum L. im Bereich von 300 mg bis 5 g, vorzugsweise im Bereich von 900 mg bis 1500 mg und/oder die Tagesdosis von Camelia sinsensis im Bereich von 200 mg bis 2500 mg, vorzugsweise von 600 mg bis 1200 mg und/oder die Tagesdosis von Curcumaextrakt im Bereich von 2 mg bis 100 mg, vorzugsweise im Bereich von 5 mg bis 30 mg und/oder die Tagesdosis des ayurvedischen Gewürzextrakts, insbesondere des Pfefferextrakts, im Bereich von 1 mg bis 20 mg, vorzugsweise im Bereich von 2 mg bis 6 mg und/oder die Tagesdosis des Bitterorangenextrakts im Bereich von 5 mg bis 50 mg, vorzugsweise im Bereich von 10 mg bis 30 mg, liegt und/oder die Tagesdosis des stimmungsaufhellend wirkenden, Scharfstoffe enthaltenden Agens mit Bestandteilen aus Zwiebel und Senf im Bereich von 10 mg bis 300 mg, vorzugsweise im Bereich von 20 mg bis 100 mg liegt.

In einer konkreten Ausgestaltung kann die Wirksubstanzmischung ggf. unter Beimengung von weiteren Träger- und Zusatzstoffen in oral einnehmbare Kapseln konfektioniert sein. Als Träger- und Zusatzstoff kommen insbesondere stoffwechselaktive MCT-Öle und/oder Sesamöl und/oder Siliziumdioxid in Betracht.

Von der vorliegenden Erfindung ebenfalls umfasst ist ein diätisches Lebensmittelset, welches eine Anzahl von Kapseln mit dem vorbeschriebenen Nahrungsergänzungsmittelpräparat, weiterhin ein Begleitpräparat zur Entwässerung, Entsäuerung und Öffnung der Fettspeicher sowie einen Mahlzeitenersatz, insbesondere in Form eines oder mehrerer Shakes umfasst.

Dabei kann das Begleitpräparat die Mineralstoffe und Spurenelemente Kalzium, Magnesium, Selen, Kupfer, Zink, Chrom und/oder Vitamin D enthalten. Weiterhin kann das Begleitpräparat mindestens einen Katalysator und/oder Wirkstoff, vorzugsweise in Pulverform, enthalten, der die Resorption der Mineralstoffe und/oder Spurenelemente steigert und/oder verbessert und/oder beschleunigt.

Der Mahlzeitenersatz in Form von Shakes kann beinhalten wenigstens ein Sojaeiweiss und/oder Milcheiweiss, wenigstens einen Mikrofaser-Ballaststoff, wenigstens einen Kohlenhydratlieferanten, wenigstens einen Fettsäurelieferanten, wenigstens ein fettlösliches und wenigstens ein wasserlösliches Vitamin, wenigstens einen Mineralstoff und wenigstens einen Geschmacksstoff.

Der Mahlzeitenersatz kann in einer bevorzugten Ausgestaltung weiterhin Isoflavone enthalten, wobei die Isoflavone besonders bevorzugt vorgesehen werden können, wenn der Mahlzeitenersatz Sojaeiweiss enthält. Der Mahlzeitenersatz in Form eines oder mehrerer Shakes ist somit speziell für eine wirksame Gewichtsabnahme durch kalorienreduzierte Mahlzeiten mit Unterstützung der Thermogenese (Umwandlung von Kalorien in Wärme), aber auch für eine sichere und gesunde Gewichtsabnahme durch die Ausgewogenheit der wertvollen und lebenswichtigen Nähr- und Vitalstoffe konzipiert.

Hochwertiges pflanzliches Sojaeiweiß mit einer sehr hohen biologischen Wertigkeit hilft die stoffwechselaktive und formgebende Muskulatur zu erhalten und damit die Gewichtsabnahme dauerhaft zu sichern. Der Mahlzeitenersatz in Form eines oder mehrerer Shakes ist zudem frei von Milcheiweiß. Weiterhin ist der Mahlzeitenersatz lactosefrei. Dadurch ist es auch für entsprechende Allergiker verträglich.

In einer alternativen Ausführung ist die Beimengung von Milcheiweiss vorgesehen, und zwar entweder zusätzlich zu Sojaeiweiss oder ausschließlich. Milcheiweiss stellt bekanntermaßen auch eine sehr hochwertige Eiweißquelle dar, insbesondere im Hinblick auf die Bildung von Muskulatur. Der Aufbau von Muskulatur ist sinnvoll, um in effektiver Weise Fett zu verbrennen.

Die Mikrofaser-Ballaststoffe bremsen den Heißhunger über einen verlangsamten Zuckereinstrom ins Blut und sorgen zudem für eine Entgiftung und eine bessere Verdauung. Die Kohlenhydrate garantieren Leistungsfähigkeit während der Gewichtsabnahme auch für das Gehirn und das Nervensystem. Darüber hinaus liefert das diätetische Lebensmittel lebensnotwendige Fettsäuren, Vitamine und Mineralstoffe. Mit Hilfe der Geschmacksstoffe werden verschiedene Geschmacksrichtungen erzeugt, so dass eine Abwechslung bei der Einnahme des Mahlzeitenersatzes entstehen kann und darüber hinaus für jede Person die richtige Geschmacksrichtung angeboten werden kann.

Die fakultativ beimengbaren und bei Shakes mit Sojaeiweiss vorzugsweise vorgesehenen Isoflavone sind sekundäre Pflanzenstoffe und wirken als Phytohormone (Phytoöstrogene). Durch den Zusatz von Isoflavonen können unter anderem Wechseljahrsbeschwerden verringert, gesunde stabile Knochen gefördert sowie Osteoporose und Brustkrebs vorgebeugt werden. Darüber hinaus können Isoflavone im Zusammenhang mit dem Cholesterinspiegel den LDL-Wert erniedrigen und den HDL-Wert erhöhen sowie das Prostata-Krebs-Risiko beim Mann reduzieren. Isoflavone wirken Hormonspiegel normalisierend und dienen als Herz-Kreislauf-Schutz.

Der Mahlzeitenersatz in Form eines oder mehrerer Shakes greift speziell beim Fettübergewicht an, das längerfristig mobilisiert werden muss. Es wird empfohlen, ein bis zwei Mahlzeiten täglich durch den Mahlzeitenersatz in Form eines oder mehrerer Shakes zu ersetzen.

Insbesondere vorteilhaft ist es, wenn der Isoflavongehalt zwischen 10 mg/100 g Mahlzeitenersatz und 200 mg/100 g Mahlzeitenersatz, vorzugsweise zwischen 30 mg/100 g Mahlzeitenersatz und 60 mg/100 g Mahlzeitenersatz, insbesondere bei 33 mg/100 g Mahlzeitenersatz, liegt. Hinsichtlich weiterer Einzelheiten in Bezug auf den hier vorgeschlagenen Mahlzeitenersatz wird auf die EP 1 588 659 A2 der Anmelderin verwiesen.

Gemäß einer beispielhaften Ausführungsform des hier vorgeschlagenen Nahrungsergänzungsmittelpräparats kann die Wirksubstanzmischung wie folgt zusammengesetzt sein:
Bockshornkleesamen-Konzentrat (Trigonella foenum-graecum L.): 1.413 mg
davon Ballaststoffe: 1.110 mg
Grüntee-Extrakt (Camelia sinsensis): 492 mg
davon Polyphenole: 246 mg
davon Coffein: 39 mg
Curcumaextrakt: 20 mg
ayurvedischer Aromaextrakt, Pfefferextrakt: 5 mg
Stimmungsaufhellend wirkendes, Scharfstoffe enthaltendes Agens mit Bestandteilen aus Zwiebel und Senf: 70 mg
davon Zwiebel: 43,2 mg
Senf: 12,8 mg
Ingwer: 14,0 mg.

Mit dem hier vorgeschlagenen Nahrungsergänzungsmittelpräparat wird eine einen Diäterfolg unterstützende Wirksubstanzmischung vorgeschlagen, die physiologische Wirkmechanismen zur Gewichtsreduzierung bzw. zum Fettabbau unterstützt und die gleichzeitig eine für den Diäterfolg wesentliche positive Grundstimmung durch ein spezielles stimmungsaufhellend wirkendes, Scharfstoffe enthaltendes Agens mit Bestandteilen aus Zwiebel und Senf schafft.

## Patentansprüche

1. Nahrungsergänzungsmittelpräparat mit einer Wirksubstanzmischung, wobei die Wirksubstanzmischung enthält:
- Trigonella foenum-graecum L. (Bockshornkleesamen),
- Camelia sinsensis mit einem Anteil von Polyphenolen und Coffein,
- Ayurvedisches Gewürzextrakt, insbesondere Pfefferextrakt sowie
- ein stimmungsaufhellend wirkendes, Scharfstoffe enthaltendes Agens mit Bestandteilen aus Zwiebel und Senf.

2. Nahrungsergänzungsmittelpräparat nach Anspruch 1,
**dadurch gekennzeichnet, dass**
das stimmungsaufhellend wirkende, Scharfstoffe enthaltende Agens mit Bestandteilen aus Zwiebel und Senf in einem Anteil von 2 Gew. % bis 20 Gew. %, vorzugsweise in einem Anteil von 4 Gew. % bis 15 Gew. %, in der Wirksubstanzmischung enthalten ist.

3. Nahrungsergänzungsmittelpräparat nach einem der Ansprüche 1 oder 2,
**dadurch gekennzeichnet, dass**
Trigonella foenum-graecum L. in einem Anteil von 15 Gew. % bis 90 Gew. %, vorzugsweise in einem Anteil von etwa 65 Gew. % bis 80 Gew. %, in der Wirksubstanzmischung enthalten ist.

4. Nahrungsergänzungsmittelpräparat nach einem der Ansprüche 1 bis 3,
**dadurch gekennzeichnet, dass**
Camelia sinsensis mit einem Anteil von 5 Gew. % bis 50 Gew. %, vorzugsweise mit einem Anteil von 20 Gew. % bis 30 Gew. %, in der Wirksubstanzmischung enthalten ist.

5. Nahrungsergänzungsmittelpräparat nach einem der Ansprüche 1 bis 4,
**dadurch gekennzeichnet, dass**
der ayurvedische Gewürzextrakt, insbesondere der Pfefferextrakt, mit einem Anteil von 0,05 Gew. % bis 0,8 Gew. %, vorzugsweise mit einem Anteil von 0,15 Gew. % bis 0,4 Gew. %, in der Wirksubstanzmischung enthalten ist.

6. Nahrungsergänzungsmittelpräparat nach einem der Ansprüche 1 bis 5,
**dadurch gekennzeichnet, dass** die Wirksubstanzmischung weiterhin enthält:
- Curcumaextrakt und/oder
- Bitterorangenextrakt.

7. Nahrungsergänzungsmittelpräparat nach Anspruch 6,
**dadurch gekennzeichnet, dass**
Curcumaextrakt mit einem Anteil von 0,2 Gew. % bis 2,5 Gew. %, vorzugsweise mit einem Anteil von 0,7 Gew. % bis 1,3 Gew. %, in der Wirksubstanzmischung enthalten ist.

8. Nahrungsergänzungsmittelpräparat nach Anspruch 6 oder 7,
**dadurch gekennzeichnet, dass**
Bitterorangenextrakt mit einem Anteil von 0,2 Gew. % bis 4,0 Gew. %, vorzugsweise mit einem Anteil von 0,6 Gew. % bis 2,0 Gew. %, in der Wirksubstanzmischung enthalten ist.

9. Nahrungsergänzungsmittelpräparat nach einem der Ansprüche 1 bis 8,
**dadurch gekennzeichnet, dass**
in dem stimmungsaufhellend wirkenden, Scharfstoffe enthaltenden Agens mit Bestandteilen aus Zwiebel und Senf
- Zwiebel mit einem Anteil von 30 Gew. % bis 70 Gew. %, vorzugsweise mit einem Anteil von 60 Gew. % bis 67 Gew. % und
- Senf mit einem Anteil von 20 Gew. % bis 50 Gew. %, vorzugsweise mit einem Anteil von 35 Gew. % bis 40 Gew. % enthalten sind.

10. Nahrungsergänzungsmittelpräparat nach einem der Ansprüche 1 bis 9,
**dadurch gekennzeichnet, dass**
es insbesondere für eine Bereitstellung in Kapseln so konfektioniert ist, dass die Tagesdosis für Trigonella foenum-graecum L. im Bereich von 300 mg bis 5 g, vorzugsweise im Bereich von 900 mg bis 1500 mg und/oder die Tagesdosis von Camelia sinsensis im Bereich von 200 mg bis 2500 mg, vorzugsweise von 600 mg bis 1200 mg und/oder die Tagesdosis von Curcumaextrakt im Bereich von 2 mg bis 100 mg, vorzugsweise im Bereich von 5 mg bis 30 mg und/oder die Tagesdosis des ayurvedischen Gewürzextrakts, insbesondere des Pfefferextrakts, im Bereich von 1 mg bis 20 mg, vorzugsweise im Bereich von 2 mg bis 6 mg und/oder die Tagesdosis des Bitterorangenextrakts im Bereich von 5 mg bis 50 mg, vorzugsweise im Bereich von 10 mg bis 30 mg, liegt und/oder die Tagesdosis des stimmungsaufhellend wirkenden, Scharfstoffe enthaltenden Agens mit Bestandteilen aus Zwiebel und Senf im Bereich von 10 mg bis 300 mg, vorzugsweise im Bereich von 20 mg bis 100 mg liegt.

11. Nahrungsergänzungsmittelpräparat nach einem der Ansprüche 1 bis 11,
**dadurch gekennzeichnet, dass**
die Wirksubstanzmischung ggf. unter Beimengung von weiteren Träger- und Zusatzstoffen, insbesondere unter Beimengung von stoffwechselaktivem MCT-Öl und/oder von Sesamöl und/oder Siliziumdioxid, in oral einnehmbare Kapseln konfektioniert wird.

12. Diätisches Lebensmittelset umfassend:
- eine vorbestimmte Anzahl von Kapseln mit dem Nahrungsergänzungsmittelpräparat nach einem der Ansprüche 1 bis 12,
- ein Begleitpräparat zur Entwässerung, Entsäuerung und Öffnung der Fettspeicher sowie
- einen Mahlzeitenersatz in Form eines oder mehrerer Shakes.

13. Diätisches Lebensmittelset nach Anspruch 13,
**dadurch gekennzeichnet, dass**
das Begleitpräparat die Mineralstoffe und Spurenelemente Kalzium, Magnesium, Selen, Kupfer, Zink, Chrom und Vitamin D enthält und dass das Begleitpräparat weiterhin mindestens einen Katalysator und/oder Wirkstoff, vorzugsweise in Pulverform, enthält, der die Resorption der Mineralstoffe und/oder Spurenelemente steigert und/oder verbessert und/oder beschleunigt.

14. Diätisches Lebensmittelset nach Anspruch 14,
**dadurch gekennzeichnet, dass**
der Mahlzeitenersatz in Form von Shakes beinhaltet:
- wenigstens ein Sojaeiweiss und/oder ein Milcheiweiss,
- wenigstens einen Mikrofaser-Balaststoff,
- wenigstens einen Kohlenhydratlieferanten,
- wenigstens einen Fettsäurelieferanten,
- wenigstens ein fettlösliches und wenigstens ein wasserlösliches Vitamin,
- wenigstens einen Mineralstoff und
- wenigstens einen Geschmacksstoff.
